Europäisches Patentamt

(19) European Patent Office

Office européen des brevets

(11) EP 0 858 989 A1

(12) EUROPEAN PATENT APPLICATION

(43) Date of publication:
19.08.1998 Bulletin 1998/34

(51) Int. Cl.$^6$: C07C 51/265, C07C 63/26

(21) Application number: 98101739.5

(22) Date of filing: 02.02.1998

(84) Designated Contracting States:
AT BE CH DE DK ES FI FR GB GR IE IT LI LU MC NL PT SE
Designated Extension States:
AL LT LV MK RO SI

(30) Priority: 12.02.1997 JP 27950/97
01.12.1997 JP 330283/97

(71) Applicant:
MITSUI CHEMICALS, INC.
Tokyo (JP)

(72) Inventors:
• Miyata, Haruo
Waki-cho, Kuga-gun, Yamaguchi-ken (JP)

• Okada, Yasuhiko
Waki-cho, Kuga-gun, Yamaguchi-ken (JP)
• Murakami, Satoshi
Waki-cho, Kuga-gun, Yamaguchi-ken (JP)
• Taniguchi, Norio
Waki-cho, Kuga-gun, Yamaguchi-ken (JP)

(74) Representative:
Hansen, Bernd, Dr. Dipl.-Chem. et al
Hoffmann Eitle,
Patent- und Rechtsanwälte,
Arabellastrasse 4
81925 München (DE)

(54) Method of controlling the quality of terephthalic acid

(57) A method of controlling the quality of terephthalic acid comprises: detecting $CO_2$ amount contained in non-condensing components in reaction waste gas discharged from a reactor having materials necessary for producing terephthalic acid; predicting the concentration of 4CBA in a product on the basis of the detected $CO_2$ amount; and controlling reaction conditions so as to maintain the predicted concentration value of 4CBA in a suitable range.

# Description

## BACKGROUND OF THE INVENTION

Field of the Invention

The present invention relates to a method of controlling the quality of terephthalic acid. More particularly, the present invention relates to a method of controlling the quality of terephthalic acid in production steps of terephthalic acid.

Description of the Prior Art

There are some production methods of terephthalic acid, and a recent main stream thereof is a production method by liquid phase oxidation of p-xylene.

In this method, a slurry in a reactor is in a state that terephthalic acid crystals is suspended in an acetic acid solution. The acetic acid solution is prepared by dissolving, in a solvent, a catalyst comprising bromine or a heavy metal, such as manganese or cobalt. And supplying p-xylene or molecular oxygen-containing gas, such as air is supplied continuously into the reactor to oxidize p-xylene, thereby forming terephthalic acid.

In this method, a large portion of terephthalic acid precipitates in the acetic acid solvent as crystals. As a result, terephthalic acid slurry is formed.

The terephthalic acid slurry formed is continuously discharged from the reactor, and then passed through the prescribed aftertreatments (crystallization step, separation/washing steps, drying step, and the like), and a powdery terephthalic acid is formed as a product.

Concentration of 4-carboxybenzaldehyde (hereinafter referred to as "4CBA") contained in terephthalic acid is one of important factors determining the quality of terephthalic acid.

The concentration of 4CBA is influenced by reaction conditions, such as reaction temperature, pressure, amount of molecular oxygen-containing gas, supplying amount of raw materials, and a retention time. In the conventional techniques, the concentration of 4CBA has been controlled by directly measuring the concentration of 4CBA in terephthalic acid, and adjusting the reaction conditions on the basis of the measurement result, so as to fall within a desired range.

However, the terephthalic acid slurry in the oxidation reactor is an acidic slurry at a high temperature and high pressure. Therefore, it is difficult to conduct sampling for measuring the concentration of 4CBA. For this reason, conventional techniques have had the only way that the terephthalic acid slurry is sampled from the portion of terephthalic acid which has passed through the precipitation and washing after oxidation reaction.

Further, the concentration of 4CBA contained in terephthalic acid is measured by using a liquid chromatography, and it takes 40 minutes or more for such a measurement. Therefore, the analytical result obtained by the sampling only shows a state of the terephthalic acid slurry inside of the reactor before several hours, and does not show the present state, which is required, of the terephthalic acid slurry inside of the reactor.

Thus, the conventional techniques require at least several hours to obtain the analytical result after controlling the concentration of 4CBA contained in terephthalic acid so as to fall within a desired range.

It may take several tens of hours, and in some cases, one day or more, in order to stabilize the inside of the reactor.

Furthermore, if there is disturbance other than operations, it takes much time in order to stabilize the inside of the reactor, and there is a fear that terephthalic acid as a product having a desired quality may not be obtained.

Even if the sampling is conducted in the oxidation reactor or at the site immediately after the reactor in the line of the production steps of terephthalic acid, and the concentration of 4CBA is measured from the sampling, the measurement time cannot be shortened. Therefore, it is merely possible to grasp only the state of the terephthalic acid slurry in the reactor before 30 to 60 minutes, and a timely control of the reaction conditions cannot be made.

## SUMMARY OF THE INVENTION

The present invention has been made to overcome the above-described problems in the prior art, and therefore an object of the present invention is to provide a method of controlling the quality of terephthalic acid, which enables obtaining terephthalic acid having a stabilized quality by grasping the concentration of 4CBA contained in terephthalic acid in a short period of time prior to obtaining terephthalic acid as a product, thereby making it possible to perform a timely control of the reaction conditions on the basis of the concentration.

The present inventors have noted a waste gas which quickly reflects the state of a reactor and is easy to conduct the sampling of terephthalic acid slurry in the reactor. As a result of extensive investigations, it has been found that there is a constant relationship between $CO_x$ concentration at which concentration measurement can be easily conducted, and the concentration of 4CBA in terephthalic acid.

As a result, there has been established a method which can predict the concentration of 4CBA by measuring $CO_x$ concentration based on the relationship, and also can control a product quality.

That is, the method of controlling the quality of terephthalic acid in its production steps according to the present invention comprises: detecting $CO_x$ amount contained in non-condensing components in a reaction waste gas discharged from an oxidation reactor having materials necessary for the production of terephthalic acid; predicting the concentration of 4CBA in a product based on the detected $CO_x$ amount; and controlling

reaction conditions so as to maintain the predicted concentration value of 4CBA in a predetermined range.

The term "reaction conditions" used herein means factors that influence the concentration of 4CBA, such as reaction temperature, pressure, amount of molecular oxygen-containing gas, supplying amount of raw materials, and a retention time.

$CO_x$ means carbon monoxide or carbon dioxide. The amount of $CO_x$ generally means the total amount of those, but may be substituted by the total amount of $CO_2$.

As the reaction conditions to be controlled, at least the reaction temperature may be changed.

Also, as the reaction conditions to be controlled, at least the reaction temperature and feeding rate of oxidation catalyst may be changed.

It is suitable for prediction of the concentration of 4CBA to use, for example, the following calculation equation in which detection amount of $CO_2$ contained in non-condensing components in reaction waste gas is a variable (parameter).

$$Y = a \cdot x + b$$

wherein

y:    Concentration (wt%) of 4CBA in terephthalic acid
a:    Regression coefficient
x:    Concentration (vol%) of $CO_2$ contained in non-condensing components in reaction waste gas
b:    Regression constant

BRIEF DESCRIPTION OF THE DRAWINGS

The objects and features of the present invention will become more apparent from the consideration of the following detailed description taken in conjunction with the accompanying drawings in which:

Fig. 1 is a schematic view showing a production line of terephthalic acid for explaining a method of controlling the quality of terephthalic acid according to the present invention; and

Fig. 2 is a view showing various means on a computer necessary for quality control.

DETAILED DESCRIPTION OF PREFERRED EMBODIMENT

Hereinafter, a method of controlling the quality of terephthalic acid according to the present invention is described in detail based on embodiments with reference to the drawings.

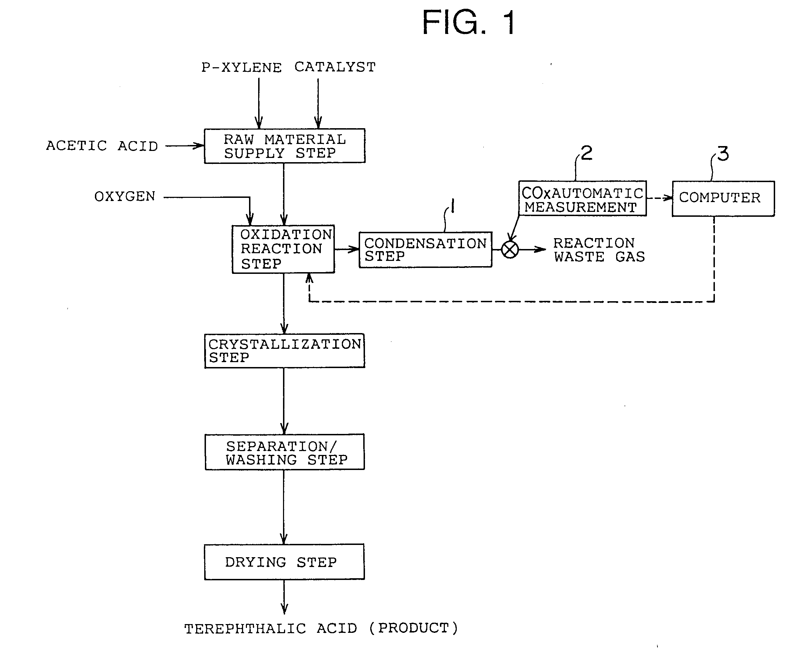

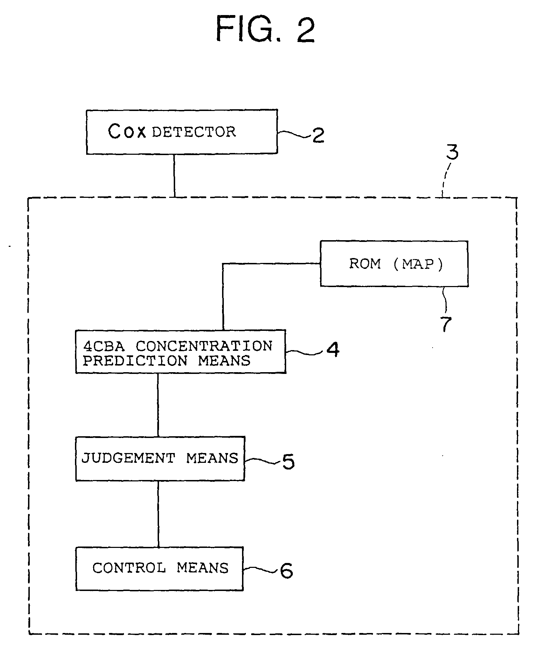

The production steps of terephthalic acid and the method of controlling the quality of terephthalic acid in its production steps are explained by referring to Fig.1 and Fig.2.

〈Outline of Production Steps of Terephthalic Acid (see Fig. 1)〉

Terephthalic acid precipitated in a slurry state by oxidation reaction step is passed through crystallization step, separation/washing steps, and drying step, thereby forming a dried powder thereof in the form of a product. There are some steps other than the recited steps, which are omitted from the explanation herein.

〈Method of Controlling the Quality of Terephthalic Acid in Production Steps of Terephthalic Acid (See Fig.1 and Fig.2)〉

Reaction waste gas line 1 after condensing reaction waste gas discharged from an oxidation reactor is equipped with a $CO_x$ concentration detector 2 which automatically detects the concentration of carbon monoxide or carbon dioxide (hereinafter referred to as "$CO_x$" in a general term) contained in non-condensing components in the reaction waste gas. The $CO_x$ concentration in oxidation step is detected by the $CO_x$ concentration detector 2. The $CO_x$ concentration detector 2 includes, for example, infrared gas analyzer or process gas chromatograph. In general, the $CO_x$ concentration detector 2 automatically continuously measures $CO_x$ concentration.

The $CO_x$ concentration detector is connected to a computer 3.

The computer 3 is equipped with 4CBA concentration prediction means 4, judgement means 5 for judging whether or not the 4CBA concentration predicted by the 4CBA concentration prediction means 4 is within a suitably predetermined range, and control means for controlling reaction conditions based on the judgement result by the judgement means 5.

The concentration of 4CBA is obtained by the prediction means 4 on the basis of the $CO_x$ concentration detected in the oxidation step. In this embodiment, $CO_x$ is found to be carbon dioxide, $CO_2$. Further, the prediction means 4 is equipped with a ROM (READ ONLY MEMOLY) 7 storing the correlation equation between the 4CBA concentration and the carbon dioxide concentration.

The correlation equation stored in the ROM 7 is prepared by a statistical technique based on the surveyed value, and includes, for example, the following equation (1) shown by a linear expression:

$$y = a \cdot x + b \qquad (1)$$

wherein

y:    Concentration (wt%) of 4CBA in terephthalic acid
a:    Regression coefficient
x:    Concentration (vol%) of $CO_2$ contained in non-condensing components in reaction waste gas

b:    Regression constant

Regression analysis when y is an objective variable and x is a predictor variable generally includes linear regression analysis, multi-order regression analysis, and polynomial regression analysis. Further, precision of the regression equation increases as the order increases.

Specific numeral values are put in the equation (1).

The regression coefficient (a) and the regression constant (b) were obtained as, for example, -0.5 and 1.1, respectively, by actually measuring $CO_2$ concentration and 4CBA. Incidentally, the data number (n) and the correlation function (r) in this case were 200 and 0.92, respectively.

Considering those, the equation (1) is expressed as the following equation (1'):

$$y = -0.5 \cdot x + 1.1 \qquad (1')$$

From the data number (n) and the correlation function (r), it is apparently understood that there is a correlation between the concentration of 4CBA in terephthalic acid and the $CO_2$ concentration. In other words, there is a negative correlation such that if the concentration of one side increases, the concentration of other side decreases.

It should be noted that the values of the regression coefficient (a) and the regression constant (b) vary depending on the difference in the production amount of terephthalic acid. Therefore, the regression coefficient and the regression constant in the equation are different every optional production amount of terephthalic acid, and it is preferable to apply the desired numeral values of those coefficient and constant to an operation expression according to the production amount of terephthalic acid.

Further, actual measurement values vary according to the measurement conditions every day. Therefore, the above equation (1) is not always applied to all the cases. However, on the assumption to be the same conditions, if the value of x is found, that is, if the concentration of $CO_2$ is found, the concentration of 4CBA can necessarily be calculated by the above equation.

Therefore, an operation expression as shown in, for example, the equation (1) is previously determined, and the operation expression is provided as a prediction means 4 realized on a computer as shown in Fig. 2.

If the detected concentration value of $CO_2$ is applied to the equation (1), the concentration of 4CBA is immediately obtained. The judgement means 5 judges whether or not the concentration value of 4CBA obtained is maintained in a suitable range (predetermined range) for terephthalic acid.

In this case, if the measurement result is outside the predetermined range, the reaction conditions, such as reaction temperature, pressure, amount of molecular oxygen-containing gas, supplying amount of raw materials, and a retention time are controlled by a control means 6. By such a control, the quality of terephthalic acid can always be controlled in the preferable state.

Incidentally, the following method is generally known as a method of controlling the concentration of 4CBA in the product terephthalic acid.

Control by oxidation reaction conditions includes, for example, adjustment of concentration of reactor, pressure, catalyst concentration, a retention time, concentration of acetic acid solution (solvent), and amount of molecular oxygen-containing gas supplied. In the conventional techniques, it was impossible to grasp the 4CBA concentration in the reactor in real time, and therefore, the respective reaction conditions were changed and controlled according to the experience. It has been found that due to the calculation of the 4CBA concentration in real time, it is preferable to use at least the reaction temperature in order to control the concentration change of 4CBA, which is the important factor for determining the quality of terephthalic acid, in a standard range, and also control having good response can be achieved by adjusting the reaction temperature and catalyst concentration. It has also be found that it is preferable for the adjustment of catalyst concentration in the reactor to change feed rate of the catalyst.

According to the above-described method of controlling the quality of terephthalic acid, it is possible to detect $CO_2$ amount contained in non-condensing components in reaction waste gas discharged from a reactor, to predict the concentration of 4CBA in a product on the basis of the $CO_2$ amount detected, and control the reaction conditions so as to maintain the predicted concentration of 4CBA in the predetermined range. Therefore, if $CO_2$ is detected, the concentration of 4CBA contained in terephthalic acid can be found. If the concentration of 4CBA is not within the predetermined range intended by the manufacturer, the reaction conditions can be controlled so as to maintain the concentration in the predetermined range. As a result, the quality of terephthalic acid can be maintained in the preferable state.

Further, since detection of the concentration of $CO_2$ can be conducted with very ease, control of the reaction conditions can also be conducted timely.

Incidentally, if desired and necessary, an operation treatment by the conventional moving average method may be employed.

**Claims**

1.  A method of controlling the quality of terephthalic acid in the terephthalic acid production steps involving oxidation reaction, which comprises: detecting $CO_x$ amount contained in non-condensing components in reaction waste gas discharged from oxidation reactor which produces terephthalic acid; predicting concentration of 4-carboxybenzaldehyde in a product on the basis the $CO_x$ amount detected;

and controlling reaction conditions so as to maintain the predicted concentration value of 4-carboxybenzaldehyde in a predetermined range.

2.  A method according to claim 1, wherein said $CO_x$ is carbon monoxide or carbon dioxide.

3.  A method according to claim 1 or 2, wherein at least reaction temperature is changed in the reaction conditions to be controlled.

4.  A method according to claim 3, wherein further at least the supply rate of oxidation catalyst is changed in the reaction conditions to be controlled.

5.  A method according to any of claims 1 to 4, wherein the following correlation equation in which the detection amount of $CO_2$ contained in non-condensing components in reaction waste gas is used for predicting the concentration of 4CBA;

$$y = a \cdot x + b$$

wherein

y:      Concentration (wt%) of 4-carboxybenzaldehyde
a:      Regression coefficient
x:      Concentration (vol%) of $CO_2$ contained in non-condensing components in reaction waste gas
b:      Regression constant

# FIG. 1

P-XYLENE   CATALYST

ACETIC ACID → RAW MATERIAL SUPPLY STEP

OXYGEN → OXIDATION REACTION STEP → CONDENSATION STEP → ⊗ → REACTION WASTE GAS

$CO_x$ AUTOMATIC MEASUREMENT — 2

COMPUTER — 3

CRYSTALLIZATION STEP

SEPARATION/ WASHING STEP

DRYING STEP

TEREPHTHALIC ACID (PRODUCT)

# FIG. 2

```
┌─────────────────────┐
│  Cox DETECTOR       │──2
└─────────────────────┘
                              3
┌ ─ ─ ─ ─ ─ ─ ─ ─ ─ ─ ─ ─ ─ ─ ─ ─ ─ ┐
│                                       │
│                  ┌─────────────────┐  │
│                  │  ROM (MAP)      │  │
│                  └─────────────────┘  │
│                            7          │
│  ┌──────────────────────┐             │
│  │ 4CBA CONCENTRATION    │──4          │
│  │ PREDICTION MEANS      │             │
│  └──────────────────────┘             │
│                                       │
│  ┌──────────────────────┐             │
│  │ JUDGEMENT MEANS       │──5          │
│  └──────────────────────┘             │
│                                       │
│  ┌──────────────────────┐             │
│  │ CONTROL MEANS         │──6          │
│  └──────────────────────┘             │
│                                       │
└ ─ ─ ─ ─ ─ ─ ─ ─ ─ ─ ─ ─ ─ ─ ─ ─ ─ ┘
```

European Patent
Office

**EUROPEAN SEARCH REPORT**

Application Number

EP 98 10 1739

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 4 835 307 A (LINDAHL HAROLD A ET AL) 30 May 1989<br>* column 1, line 36 - column 2, line 53 *<br>* column 5, line 12 - line 50 *<br>* column 6, line 3 - line 17 *<br>* column 7, line 49 - line 51 *<br>* figure 2 *<br>--- | 1-4 | C07C51/265<br>C07C63/26 |
| X | PATENT ABSTRACTS OF JAPAN vol. 004, no. 022 (C-074), 23 February 1980<br>& JP 54 160330 A (KURARAY YUKA KK), 19 December 1979,<br>* abstract *<br>----- | 1-3 | |
| | | | TECHNICAL FIELDS SEARCHED (Int.Cl.6)<br>C07C |

The present search report has been drawn up for all claims

| Place of search | Date of completion of the search | Examiner |
|---|---|---|
| THE HAGUE | 25 May 1998 | Held, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)